Europäisches Patentamt

European Patent Office

Office européen des brevets

(11) Publication number: **0 509 328 A2**

## EUROPEAN PATENT APPLICATION

(21) Application number: **92105687.5**

(22) Date of filing: **02.04.92**

(51) Int. Cl.⁵: **G01N 29/00, G01N 29/18**

(30) Priority: **04.04.91 JP 71452/91**

(43) Date of publication of application:
**21.10.92 Bulletin 92/43**

(84) Designated Contracting States:
**DE FR GB**

(71) Applicant: **HITACHI, LTD.**
**6, Kanda Surugadai 4-chome**
**Chiyoda-ku, Tokyo 101(JP)**

(72) Inventor: **Arai, Yuko**
**3-68-A402, Shiraume 2-chome**
**Mito-shi(JP)**
Inventor: **Honda, Takashi**
**2917-24, Mukaino**
**Mawatari, Katsuta-shi(JP)**
Inventor: **Ohnaka, Noriyuki**
**2029-1, Higashiishikawa**
**Katsuta-shi(JP)**

(74) Representative: **Patentanwälte Beetz - Timpe -**
**Siegfried - Schmitt-Fumian- Mayr**
**Steinsdorfstrasse 10**
**W-8000 München 22(DE)**

(54) Method of detecting minute gas component in ambience and applied devices.

(57) To provide a method of environment monitor capable of detecting a minute gas component for monitoring an ambience, and various systems including a gas detector (1). According to the present invention, a method and various systems using the same method are provided in which a sensor for applying an ultrasonic vibration to a thin film (4) formed on a piezoelectric substrate (2) to measure the frequency change of a surface acoustic wave is used to detect a minute gas with high sensitivity. The change of a material in an ambience can be measure by use of this gas sensor.

F I G. I

## BACKGROUND OF THE INVENTION

The present invention relates to a method of detecting a minute gas component in an ambience using a novel gas sensor and an applied apparatus.

Conventionally, an example of application of a sensor utilizing a piezoelectric substrate having a interdigital transducers for input and output of a surface acoustic wave is described in JP-A-63-250561 and Collection of Speeches before Japan Acoustic Society, March 1989, pp. 679 to 680. According to them, an Au thin film is formed on a surface wave propagation surface or as interdigital transducers of a piezoelectric substrate, and a specific substance in a solution or the viscosity of a liquid is detected from the displacement of the oscillation frequency by use of a sliding surface wave parallel to the substrate surface and having a particle displacement in the direction perpendicular to the direction of propagation. The change in the oscillation frequency of the piezoelectric substrate of the sensor is detected by the surface of the surface wave propagation between the interdigital transducers due to a solution or viscosity change or by the adsorption of a specific substance to a thin film material forming the substrate, thus obtaining information depending on the solution and independing on detecting the gas component.

The above-mentioned conventional system is such that only the physical information on the solution side is detected by the surface wave propagation surface on a piezoelectric substrate and the Au thin film formed on the propagation surface fails to react with an environmental factor.

## SUMMARY OF THE INVENTION

The object of the present invention is to provide a method of detecting a minute gas component and monitoring an environment, and an apparatus having a gas detector therefor and an applied apparatus for detecting a minute gas component in an ambience with high sensitivity and measuring a change including the corrosion of a material in an environment with high sensitivity by detecting, real time with high sensitivity, a minute reaction in an environment of a foil, a film or a thin film composed of a metal, ceramics or an organic chemical compound in an intended environment formed on the surface of a piezoelectric substrate.

According to the present invention, there is provided a method for monitoring an environment or a minute gas component in an ambience characterized in that an ultrasonic vibration is caused in a thin film formed on a piezoelectric substrate and a frequency change propagated through said thin film due to the reaction between the thin film and the ambience or the minute gas component in the ambience is detected, thereby detecting the minute gas component or a material change or a corrosion behaviour affecting the material constituting the thin film in an ambience.

There is provided a method for detecting a weight change of the material constituting the thin film in said ambience or a minute gas component in said ambience, wherein said thin film is composed of at least one of a metal, an organic chemical compound and a ceramics.

According to the present invention, there is provided an apparatus comprising a gas detection device for detecting a minute gas component in an ambience, comprising a gas sensor for sensing the minute gas component, oscillation means for causing an ultrasonic vibration in said gas sensor and detector means for detecting the frequency of the surface acoustic wave generated from said gas sensor, characterized in that said gas sensor includes a piezoelectric substrate, a thin film composed of a material reactive with said minute gas component and formed on said substrate, an electrode formed on said substrate surface for causing the ultrasonic vibration and an electrode for detecting the ultrasonic vibration thus caused.

The present invention is for detecting the above-mentioned minute gas or an odor in an ambience, and characterized by a display unit for displaying a detected minute gas component or odor component in accordance with the detected value. Also, one aspect of the present invention is to provide a deodoring device for removing the odor component detected.

According to the present invention, a thin film reactive to an ambience is used as a gas detection device, and a corrosive environment of an apparatus is monitored by watching the process due to the reaction between the particular thin film and the ambience. This invention is usable not only against corrosion but also for a change in material.

A specific example of application of the present invention is characterized by a refrigerator comprising an outer box and an inner box between which a heat insulating material is filled, and a refrigeration chamber and a freezing chamber partitioned by the inner box, wherein said refrigeration chamber has therein at least a gas sensor for detecting the putrid smell of foods and a display unit for displaying the degree of putridity in accordance with the output of the gas sensor, especially outside the refrigeration chamber.

According to the present invention, there is provided an air-conditioning system for detecting a minute offensive odor in the interior air and replacing with an outside air or deodoring the interior air in accordance with the value thus detected, wherein said odor sensor includes a piezoelectric substrate and a thin film composed of a material reactive to said odor component formed on said odor sensor, an ultrasonic vibration is applied to said odor sensor by an oscillator and a frequency change of a surface acoustic wave due to the reaction between the thin film and the odor component is detected by a detector thereby to detect said offensive odor.

Other examples of application include the air freshener, the air conditioner, the microwave oven, the refrigerator, the dish washer, the dish dryer, the cleaner, the washing machine and the cloth dryer. These apparatuses are equipped with a deodorizer or the like. The sensor according to the present invention is usable also for detecting bacteria. It may be mounted on the various apparatuses described above.

The apparatus according to the present invention is capable of detecting dust and is usable as a dust sensor in a clean room. In addition, it is possible to detect harmful gases (such as $SO_2$, $H_2S$, $SO_x$ and $NO_x$) in the atmosphere, thereby permitting the monitoring against public nuisances.

According to the present invention, a surface acoustic wave is generated and detected by interdigital transducers formed on a piezoelectric substrate. On the basis of the principle that a change in oscillation frequency $\Delta f$ is proportional to $-\Delta m$, i.e., a weight change $\Delta m$ of the thin film formed on the surface wave propagation surface between interdigital transducers, multiplied by -1, the reduction in $\Delta f$ corresponds to an increased weight and the increase in $\Delta f$ to a decreased weight of the thin film.

Also, by using a wave of SH (Shear Horizontal) mode of all the surface acoustic waves, oscillation even in a solution is made possible, thereby realizing a sensor usable in both gas and liquid phases.

A piezoelectric substrate and interdigital transducers capable of generating a surface acoustic wave of SH mode is selected and a foil, a film or a reactive thin film composed of a metal, an organic chemical compound or ceramics is formed on the propagation surface of the surface acoustic wave between interdigital transducers, whereby a slight weight change of a foil, film or a reactive thin film composed of an organic chemical compound, ceramics or a metal due to the reaction with a component of an intended environment is capable of being measured continuously with a sensitivity as high as a PPb order real time in terms of a change in oscillation frequency of the surface acoustic wave. The reaction is obtained directly as a weight change due to a chemical reaction with a thin film or adsorption.

Piezoelectric substrates capable of generating a surface acoustic wave of SH mode include $LiTaO_3$, $LiNaO_3$ and crystal. Of these materials,

$LiNaO_3$ has the highest power concentration on the substrate surface, crystal has a great stability against temperature changes, and $LiTaO_3$ ranks between them for both of the properties.

Piezoelectric substrates capable of selectively generating a source acoustic wave of SH mode include 36°-rotated Y-plate X-propagation $LiTaO_3$, X-cut $LiTaO_3$, 41°-rotated Y-plate $LiNaO_3$, 64°-rotated Y-plate $LiNaO_3$, ST-cut crystal and 41°-rotated Y-plate crystal.

Input-output interdigital transducers for generating a surface acoustic wave include an Al thin film. In a harsh practical environment, however, an Au thin film is superior in durability.

In forming interdigital transducers of an Au thin film, the use of a Cr thin film as a base improves the cohesion with the piezoelectric substrate. A method suitable for forming interdigital transducers on a piezoelectric substrate is by deposition by evaporation using the heating or sputtering. In the case where the comb-shaped electrodes are not adversely affected by the deterioration or the like in a practical environment, it is possible to employ a structure, with an exposed surface containing the comb-shaped electrodes on the piezoelectric substrate and the surface acoustic wave propagation plane between the interdigital transducers. When the interdigital transducers are liable to be considerably affected adversely by the deterioration or the like in a practical environment, on the other hand, only the surface acoustic wave propagation surface between the comb-shaped electrodes is exposed, while the remaining portions are hermetically enclosed.

By appropriately selecting a reactive material formed on the plane of surface acoustic wave propagation between interdigital transducers, a sensor is made available which is capable of selectively detecting an intended component. If an enzyme film is fixed as a reactive material, and glucose oxidase, alcohol oxidase, glycerol dehydrogenase, lactate oxidase, uricase, glutamate dehydrogenase, asparaginase, methionine ammonialyase, urease, cholesterol oxidase, lipase, phospholipase, penicillinase, gliatinase or phosphatase is used as such an oxygen film, then it is possible to produce a biosensor mounted in an analytical system for a clinical laboratory capable of selectively detecting a biological component such as glucose, ethanol, glycerol, lactic acid, uric acid, glutamic acid, asparagine, methionine, urea, cholesterol, neutral lipid, phospholipide, penicillin, gliatin and phosphoric ions, respectively. A thin film reactive to a component in an environment as a reactive material, such as a synthetic film of a lipid, is formed to react with a minute component in the environment, whereby it is possible to provide a sensor for detecting the quantities of specific organic substances, various odor components or bacteria.

A minute gas component can be detected by utilizing the change due to the adsorption thereof to Teflon, polyethylene or silicon resin.

A conventional method of quantitative measurement of a biological or odor component includes a sensor using a semiconductor, or a liquid or gas chromatograph. The disadvantage of a semiconductor sensor is a low responsiveness and that of a liquid or gas chromatograph is a long time required for measurement. When the change in oscillation frequency of the surface acoustic wave of a piezoelectric substrate is used, by contrast, not only a direct continuous measurement but also a rapid measurement with a sensitivity in the order of ng is possible. An analytical system for the clinical laboratory with high sensitivity is realized by incorporating a sensor according to the present invention as a probe. Also, automation of the inspection work in the manufacturing processes where an odor is a problem is made possible. Further, a sensor having a film reactive to a specific organic substance, an odor component or bacteria, which may be formed as a reactive material on the propagation plane of a surface acoustic wave between interdigital transducers, may be mounted in an air-freshener, air-conditioner, refrigerator, microwave oven, dish washer, dish dryer, electric cleaner, electric washing machine, cloth dryer or the like home-use appliances. By doing so, the generation and the quantity of bacteria or an offensive odor in these home-use appliances themselves and also the environments including the automobiles, airplanes or ships in which they are used, are detected real time with high sensitivity. If these devices are used as a sensor for monitoring such factors, it is possible to control the time of cleaning, washing or other operations of the device used for removing the bacteria or the like, thereby improving the function of these home-use appliances, while making it usable also for measurement of the air or water pollution in outdoor or river environments.

In the case where a thin film or foil made up of ceramics or metal is used as a reactive material, the material deterioration behaviour of the particular thin film or foil in a specific environment can be monitored. When Si or $SiO_2$ is made as a thin film or foil of a reactive substance, it is possible to monitor the oxidization behaviour of Si or the reaction between a component and $SiO_2$ in an intended environment. This technique may be applied to the production of electronics parts. The use of these compounds as a reactive material for Al, Cu, Ni, Fe, Cr, Co or the like of electronics or information systems permits detection of the corrosion rate of the metals in an environment. If Ti, Zr or an alloy containing Ti, Zr is formed as a thin film or foil, the quantity of hydrogen adsorbed to the thin film or foil of Ti, Zr or the alloy containing it respectively can be measured.

In the case where only one of a thin film, a film and a foil of either a metal, an organic chemical compound or ceramics is formed as a reactive material, a single-purpose sensor is obtained. When a plurality of them in the same plane form are mounted, however, a functionary gradient material or the quantification of a component is made possible. These plurality of types of reactive material are arranged in plane form divided into a plurality of equal portions in parallel to each other and/or longitudinally between the interdigital tarnsducers.

In the case where the reaction between a reactive material and an intended component in an intended environment is used reversibly, a piezoelectric substrate can be reused by heating, electric excitation or cleaning with a wind pressure applied on the surface of the reactive material. In the case where a reactive material is reactive irreversibly with an intended component in an intended environment, on the other hand, related portions of the reactive material, the piezoelectric substrate or the sensor body are made in demountable fashion. In the process, an arrangement is made for the sensor to recognize and notify the life of the reactive material.

The use of a sensor according to the present invention makes it possible to detect the water or gas component in an environment, the minute ion concentration of a solution and the concentration of minute molecules dissolved, quantify the gas adsorption into a metal or detect the deterioration behaviour of such materials as metals and ceramics.

Also, by use of a sensor according to the present invention, a gas emanating from a food may be detected, the proper time of eating a given food may be determined from the type or the amount thereof. The sensor may thus be used as a storage of them. Further, the appropriate timing of shipment of foods or fruits can be scientifically determined correctly. Acetylene emanating in initial stages of decomposition, for example, may be used as a guide post.

Assume that a reactive thin film, a film or a foil made of a metal, a ceramics or an organic chemical compound is formed between interdigital tarnsducers for propagating a surface acoustic wave on a piezoelectric substrate with the interdigital tarnsducers arranged in opposed relation to each other for input and output of a surface acoustic wave and the thin film, the film or the foil, as the case may be, is exposed to an environment intended for measurement. The reaction with a component in an environment can be caused on the thin, film or the foil respectively. Also, by connecting a sensor having the structure described above, an oscillation circuit and a frequency counter, it is possible to grasp the components in the environment and a weight change due to the reaction of the thin film, the film or the foil as a change in oscillation frequency. Further, on the basis of the chronological change of the change amount of the oscillation frequency, the concentration of a component that has reacted in an environment as well as the rate and amount of reaction of a particular thin film, film or foil can be calculated by a fluxional analysis unit, thereby making it possible to detect the concentration of a specific component in an environment. In the sensor having the above-mentioned structure, a circuit may be inserted to detect the difference of oscillation frequency between a portion where the thin film, the film or the foil is exposed and a portion where it is not exposed, so that the frequency variation due to a temperature change of the piezoelectric substrate can be offset, thereby providing a temperature-compensated sensor.

## BRIEF DESCRIPTION OF THE DRAWINGS

These and other objects and technical advantages of the present invention will be readily apparent from the following description of the preferred exemplary embodiments of the invention in conjunction with the accompanying drawings, in which;

Fig. 1 is sectional view of a gas sensor according to an embodiment of the present invention.
Fig. 2 is plan view of the system shown in Fig. 1.
Fig. 3 is a block diagram of a gas detector using the gas sensor shown in Fig. 1.
Fig. 4 is a diagram showing the process of adsorption of water to an Au thin film as measured by a sensor according to the present invention.
Fig. 5 is plan view of a gas sensor having a temperature-compensated sensor.
Fig. 6 is a block diagram of a gas detector using a gas sensor shown in Fig. 5.

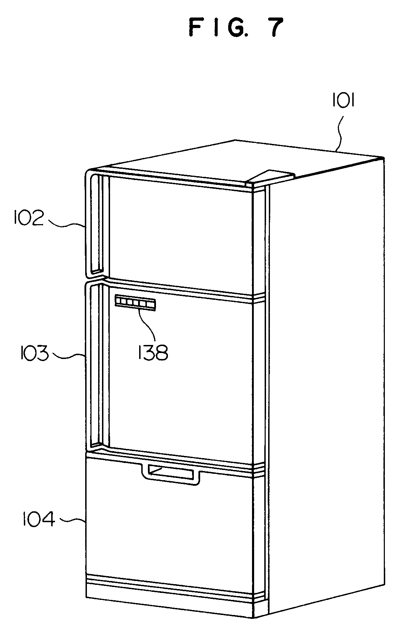

Fig. 7 is perspective view of a refrigerator using a gas sensor according to the present invention.
Fig. 8 is sectional view of the system shown in Fig. 7.
Fig. 9 is a block diagram of a gas detector used for the refrigerator shown in Fig. 8.
Fig. 10 is sectional view of a refrigerator with a gas sensor having a temperature-compensating cell.
Fig. 11 is sectional view of an air-conditioning system for the automobile having a gas sensor according to the present invention.
Fig. 12 is a diagram showing a circuit for Fig. 11.

## DETAILED DESCRIPTION OF THE PREFERRED EMBODIMENTS

### Embodiment 1

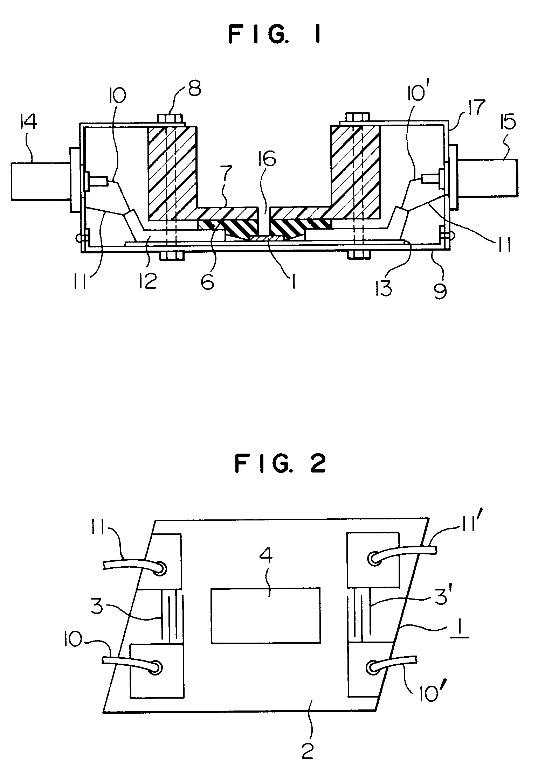

Fig. 2 shows a plan view of a gas sensor for measuring the change in a surface acoustic wave on a piezoelectric substrate according to the present invention. The sensor includes a sensing thin film on a plane of surface acoustic wave between an input interdigital transducer 3 and an output interdigital transducer 3' on a piezoelectric substrate 2. Fig. 1 is a cross sectional view of a measuring cell. The recessed part at the central section is a sensing unit having a sensing thin film 4 of the sensor 1 shown in Fig. 1, left exposed, with the remaining portions covered with fluoro rubber or silicon rubber 6, further covered with a polyacrylic sheet 7. Then a bottom plate 9 is fixed with a screw 8 for hermetic closure. This structure of hermetic enclosure for other than the sensing thin film 4 makes measurement possible even in gas phase.

Two input and output interdigital transducers have metal thin films 4 μm wide and 4 mm long arranged alternately with metal thin films similarly formed on an earth 11. Each electrode is subjected to ultrasonic bonding with lead wires 10, 10' and an earth lead wire 11 on a metal thin film of a 4 mm square. The substrate 2 forms a parallelogram cut obliquely in a size 20 mm by 14 mm wide in such a manner as to remove the effect of a reflected wave. Numerals 14 and 15 designate input and output terminals. Numeral 13 designates a wiring substrate, and numeral 16 a portion exposed to the sensing thin film 4. The earth lead wire 11 is connected to a metal case 17. The sensing thin film measures a length of 8 mm by a width of 4 mm by a thickness less than the wavelength of a propagating surface wave.

The surface acoustic wave on a piezoelectric substrate is used together with a variation of the surface wave frequency propagating between interdigital transducers caused by the elastic change. Since the detection sensitivity increases with frequency, a high-sensitivity measurement of at least QCM of less than 10 MHz and more in the order of 100 MHz is made possible, thereby permitting the detection of a gas component in the PPb order.

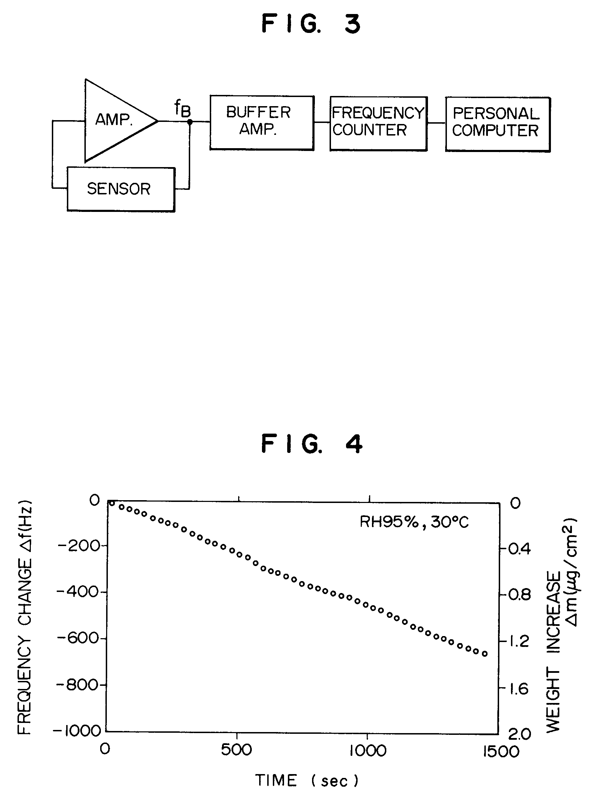

Fig. 3 shows an example of configuration of a measuring system. The sensor shown in Fig. 1 is oscillated by an oscillation circuit configured of an amplifier and a buffer amplifier, and the oscillation frequency changing with the reaction on the sensing thin film 4 is measured by a frequency counter. A frequency change Δf thus obtained is introduced as a data into a personal computer, converted into a corresponding mass change and processes to be displayed as a graph of chronologial change.

Fig. 4 shows the result of continuous measurement of the process of reaction of water adsorption to the surface of an Au thin film. This Au thin film is deposited by vacuum evaporation in the thickness of 1000 Å on the plane of surface acoustic propagation between the interdigital transducers 3, 3', which constitutes an Au-deposited film (with a Cr deposited film as a base) 2 mm in aperture, 40 $\mu$m in periodicity and 15 in finger pairs. The piezoelectric substrate 2 of the sensor 1 shown in Fig. 1 is composed of a 36°-rotated Y-plate X-propagated $LiTaO_3$. The measuring cell 5 shown in Fig. 2 is thus prepared, and the sensing thin film 4 is installed in an ambience 95% in relative humidity and 30 °C in air temperature as a measuring system configured as shown in Fig. 3. With the progress of water adsorption on the Au thin film, the value $\Delta f$ shifts to negative side in accordance with the increase in thin film weight, thereby making it possible to monitor the water adsorption behaviour to the Au thin film.

The sensitivity of this sensor is determined in terms of 2 ng/cm$^2$/Hz from a preliminary experiment, and therefore the quantity of adsorbed water after about 1500 seconds is about 1.3 $\mu$g/cm$^2$, which corresponds to a molecular weight of about 7 as a water molecule.

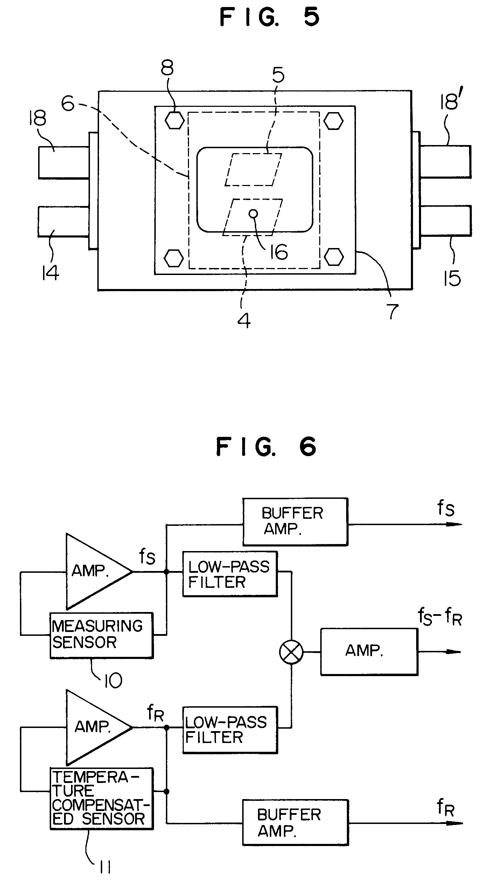

Fig. 5 shows an example of a plan view of a gas-measuring cell having a temperature compensating function. A gas-measuring sensor and a thin film 5 of a temperature-compensating sensor are formed in the same cell. Only the sensing thin film 4 of the measuring sensor 1 is exposed, and the remaining portions are covered with a silicon rubber 6. The temperature-compensating sensor is configured the same way as the gas-measuring sensor 1. The cross section of this gas-measuring cell is configured the same way as that of Fig. 1. Numerals 18, 18' designate lead-connecting terminals of the temperature-compensating sensor.

Fig. 6 is a block diagram showing a sensor circuit using the two sensors in Fig. 5. This circuit is configured in such a manner as to measure the oscillation frequencies $f_S$ and $f_R$ of a measuring sensor and a temperature-compensating sensor, and detect the difference between $f_S$ and $f_R$ for the measuring sensor and the temperature-compensating sensor respectively on the one hand and the difference between $f_S$ and $f_R$ through a double balance mixer on the other hand.

By using the sensor shown in Fig. 5 and the circuit shown in Fig. 6, the effect of temperature during the measurement is eliminated, thereby making possible a high-accuracy detection in the PPb order. The characters $f_S$ and $f_R$ in Fig. 6 designate lines for measuring the frequency which are connected to the terminals in Fig. 5. The difference between the frequency $f_S$ of the measuring sensor and the frequency $f_R$ of the temperature-compensating sensor is indicated by $f_S$-$f_R$ and is processed by an external circuit.

Embodiment 2

Fig. 2 is a perspective view showing a refrigerator having the gas-measuring cell shown in Fig. 1 as an odor sensor. As shown in the drawing, the refrigerator includes a body 101, a freezer door 102, a refrigerator door 103 and a vegetable room door 104.

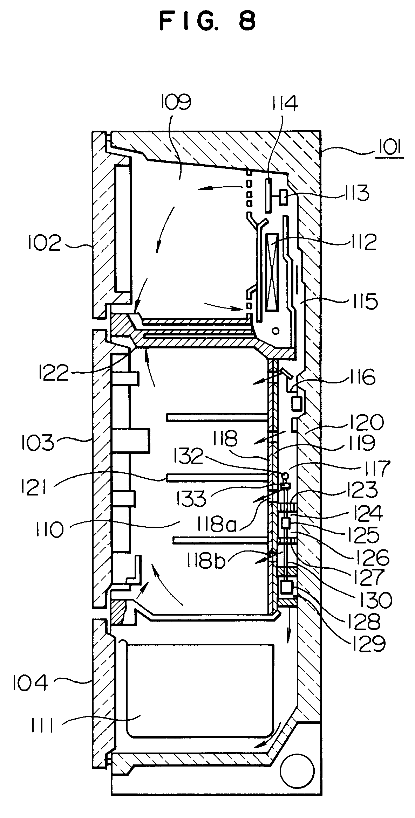

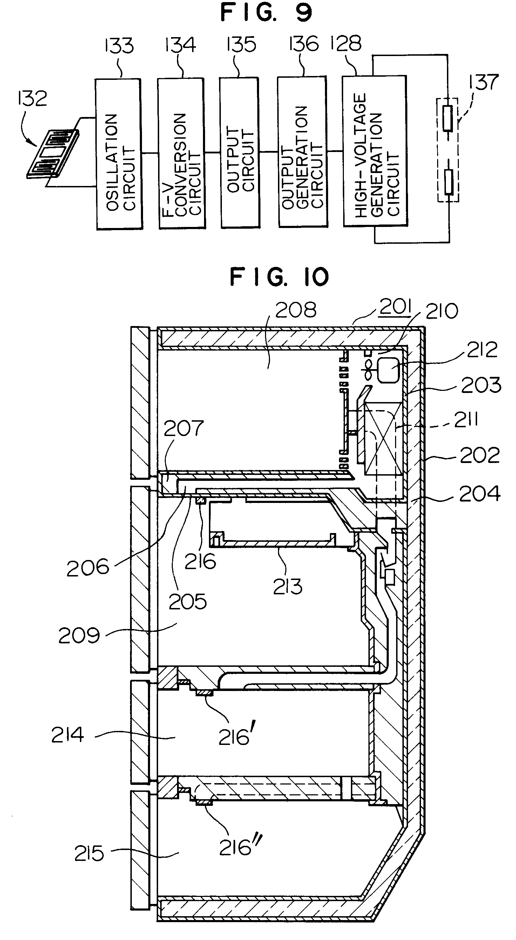

In Figs. 8 and 9, the refrigerator 101 is partitioned into a freezing chamber 109, a referigeration chamber 110 and a vegetable chamber 111. The cooler 112 is for blowing the cool air cooled by the cooler 113 into each chamber by a fan 114 driven by a cooling fan motor 113.

Part of the cool air is used to control the blowout amount of the cool air by a damper thermostat 116 installed at the outlet of a cool air duct 115 therethrough on the back of the freezing chamber 109, thereby maintaining the refrigerator interior at a constant temperature. Numeral 117 designates a duct formed of a control panel 118, a heat insulating member 119 and a back internal box 120 on the back of the refrigeration chamber 110. The control panel 118 and the heat insulating member 119 have a plurality of blowout vents 118a. The refrigeration chamber 110 has a plurality of stacks 121. The cool air is returned to the cooler 112 by a return duct 112. An ozone decomposer 123, an ozone reaction chamber 124, an ozone generator 125, an ozone reaction chamber 126 and an ozone decomposer 127 are arranged sequentially in the duct 117 and constitute a deodorizer, which is disposed between the blowout vents 118a and 118b in vertical direction. The odor sensor 132 mentioned above is disposed in the duct 117 before the ozone reaction chamber 126. There is a controller for energizing the ozone generator 125 in accordance with the output of the odor sensor 132. Further, the flat plates of the electrodes of the ozone generator 125 are mounted in the direction parallel to the flow of the cool air. The sensing portion of the sensor has, for example, a molecular film having a liposome, a phosphor lipide of lecithin or the like, cholesterol, an artificial synthetic film, a cellulose film or other LB film like arachin. The protein or the hydrogen sulfide making up a putrid odor of protein, indol, methyl mercaptan or the like sulfur component, and the fish and other offensive odors of putrid fish composed of an amine component like ammonium, ethyl amine or trimethyl amine is detected in minute amount in the PPb order to the same degree as felt by the human body. Also, apart from the ozone generation from an ozone-generating electrode, a display unit 138 for notifying the degree of putridity in accordance with the detection output of the odor sensor 132 is mounted on the referigerator doors 103, 104 for indication in a plurality of stages in the form of bar graph.

In order to apply a high voltage to the ozone generator 125, a high-voltage generator 128 is disposed in an accommodation section 129 patitioned from the duct 117 by a partition wall 119 integrated with the heat-insulating material 19 below a cool air blow-out vent 18b at the bottom. The accommodation section 129 is sealed to cool air between the partition wall 119a and the inner box 120 to keep the high-voltage generator 128 from water.

The high-voltage generator 128 and te ozone generator 125 are connected to each other by a high-voltage cord 130.

The cool air cooled by the cooler 112 is blown into the duct 107 by the fan 114 through the cool air duct 115 and the damper thermostat 116. The cool air thus blown into the duct 117 is supplied to the refrigeration chamber 210 from the cool air vents 118a, 118b of the the control panel 118 and the duct member 119 covering the duct 117 through the ozone decomposer 124, the ozone reaction chamber 124, the ozone generator 125, the ozone reaction chamber 126 and the ozone decomposer 127.

A high voltage generated by the high-voltage generator 128 is applied to the ozone generator 125 and the ozone thus generated is filled in the ozone reaction chambers 124, 126.

When the cool air containing the component of an offensive odor of a food in the refrigerator passes through the duct 117, the offensive odor component is detected by the odor sensor 132, and in accordance with the detected concentration, a notice warning against food handling in the refrigerator is displayed on the front panel of the refrigerator. At the same time, a controller 133 sends a signal to the ozone generator in such a manner as to generate ozone in an amount commensurate with the detected concentration. The ozone thus generated is supplied to the ozone reaction chambers 124, 126 thereby to decompose the offensive odor component by reaction with ozone. The extraneous ozone generated is decomposed in ozone decomposers 123, 127, and therefore even when ozone flows out into the refrigerator 210, there is posed no problem of ozone odor or discoloration of plastics parts. When the damper thermostat 116 is opened and the cool air is flowing in the duct 117, the direction of the cool air flow is downward. As long as the damper thermostat is closed, however, the cool air may flow upward due to the convection of the cool air. For this reason, the ozone decomposers 123, 127 are installed above and below the ozone generator 125.

The cool air of which the offensive odor is decomposed returns to the cooler 112 through a return duct 122 and is circulated after cooling the refrigerator interior, thereby deodorizing and cleaning the cool air for the entire refrigerator interior.

Explanation was made above about the circulation and deodorization of the cool air in a 3-door refrigerator. Regardless of the number of doors, however, the present invention is applicable to the circulation of cool air and deodorization of other chambers including the vegetable chamber and the like. Apart from a high-voltage generator and an ozone generator as a deodorizer, the system according to the invention may use an ultraviolet ray lamp and a ballast with equal effect. Also, instead of installing a plurality of cool air vents and a plurality of ozone decomposers and ozone reaction chambers as in the example shown above, it is possible to arrange a plurality of series of an ozone generator, an ozone reaction chamber and an ozone decomposer sequentially with a high-voltage generator in a duct.

According to this embodiment, there is provided a refrigerator for cooling an air forcibly by a fan, in which an ozone decomposer, an ozone reaction chamber, an ozone generator, an ozone reaction chamber and an ozone decomposer are arranged in that order to form a deodorizer in a duct for leading the cool air into a refrigeration chamber, thereby saving the space. Further, a display unit (EL unit) may be installed for displaying the generation of an offensive odor in several stages in accordance with the concentration thereof on a refrigerator door 103. This makes it possible to detect the degree of putridity in the refrigerator.

Fig. 9 is a circuit diagram showing an ozone generator comprising an oscillation circuit 133, an F-V conversion circuit 134, an output circuit 135, an output generator circuit 136 for generating an output voltage to turn on and off according to the magnitude of voltage by an output, and a high-voltage generator circuit for generating and applying a high voltage to an ozone generating electrode 137 when the output is on. Specifically, the concentration of an offensive gas of the deodorizer mentioned above is detected by the odor sensor 132, and in accordance with the detection output of the gas sensor associated with a predetermined or higher concentration of the offensive gas, the ozone generating electrode is turned on and off. The ozone quantity is thus always maintained constant.

As described above, according to the present embodiment, a box is constructed to pass the cool air using an odor sensor very high in sensitivity, and an ozone generator is installed in the path thereof for generating an ozone for decomposing an offensive odor. On the other hand, a catalyst filter for adsorbing the offensive odor and reducing it to oxygen is disposed at the outlet. Also, a gas sensor for detecting the concentration of an offensive odor is arranged in proximity to the ozone generating electrode. Further, a deodorizer with an electrical circuit device in the above-mentioned box for controlling the time of energization of the ozone electrode in accordance with the detection output of the gas sensor is installed at an appropriate position in the cool air path in the refrigerator. As a result, a refrigerator is provided which maintains an always fixed quantity of ozone and a high deodorization function for long time, free of such maintenance requirements as replacement of the deodorizing agent.

Embodiment 3

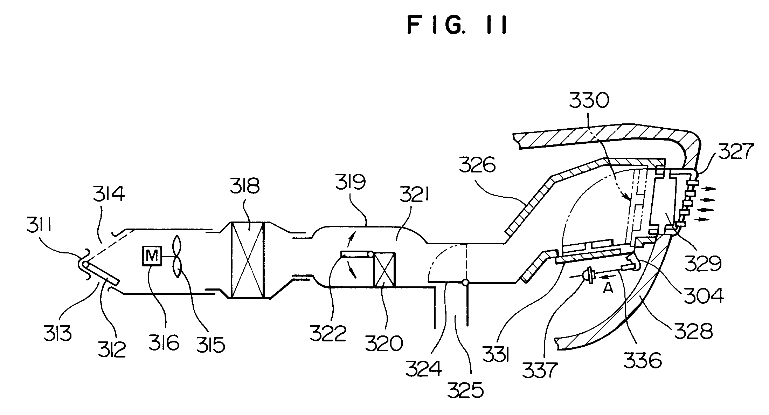

Fig. 10 is a sectional view of a refrigerator comprising an odor sensor and a display unit therefor according to the present invention. 201 designates a refrigerator body including an outer box 202, an inner box 203 and a foamed insulating member 204 filled in between the boxes 202, 203. Numeral 205 designates a partition wall formed integrally with the inner box 203. The partition wall has a foamed styrol insulating member 207 inside thereof with a freezing chamber 208 at the upper part and a refrigeration chamber 209 at the lower part thereof partitioned from each other. 210 designates a cooling chamber formed behind the freezing chamber 208, which has built therein a cooler 211 and a blower 212 for forcible ventilation. 213 designates a deodorizer installed at a corner between the side wall and the partition wall 207 in the ceiling of the refrigeration chamber. The deodorizer has a high-voltage generator, an ozone generator, an ozone decomposer (none of which is shown) and a blower therein.

The odor sensors 216, 216', 216'' according to the present invention include a temperature-compensated sensor explained with reference to embodiments. Except for the freezing chamber 208, a display unit is arranged at the upper part of each door of the refrigeration chambers 209, 214 and 215 for displaying the detected concentration in several stages.

According to the present embodiment, the deodorizer is manually operable watching the concentration displayed on the display unit. The conditions of raw foods can be determined from the display. Especially, the odor sensors 216, 216' and 216'' have a thin film of an organic chemical compound for sensing a gas generated in accordance with the foods in each refrigeration chamber. It is specifically recommended that the sensor functions be divided into meat and vegetables applications.

Acetylene gas emanates in initial stages of putridity of vegetables, and the use of a sensor for detecting it would make fresh foods available. In a refrigerator, a sensor according to the present invention is installed in a meat-fish storage and a vegetable storage, and the freshness of meat and fish and vegetables is determined (by a freshness sensor) from the amount of the generated gas composed of amine components and the like. In the case where the value exceeds a certain tolerance, the proper timing of eating or a forecast of putridity is notified in stages by a display on the front panel of the refrigerator door. For example, the proper timing of eating vegetables or fruits is displayed in blue, a warning against the degree of putridity of meat or fish in yellow, and a recommended time of disposal in red to call the attention of the user. As an alternative, the same color in different shades may be used for notification of different timings.

Even aged persons who have a deteriorated olfactory sense can place their foods under safe control. For this application, the sensing portion of the sensor is formed of, for example, a phospholipid such as a molecular film recithin having liposome, cholesterol, artificial synthesetic film, cellulose film, arachic acid or the like LB film.

Further, in a refrigerator, the problem is posed by the generation of an offensive odor composed of protein or hydrogen sulfide of putrid protein, sulfur components such as indole and methyl mercaptan, or amine components like ammonium, ethyl amine and trimethyl amine causing odors of fish or putridity thereof. In view of this, the sensor described above is installed in the meat-fish storage and the vegetable storage, so that an offensive odor is detected and the generation of an offensive odor or putridity is notified on a display on the basis of the amount of a detected offensive odor. A recommended method of display consists in a bar graph by color for indicating the degree of freshness in stages on the refrigerator door by means of a liquid crystal or EL device. Such a system is adapted to energize a deodorizer or a deodorization system when a tolerance is exceeded.

Embodiment 4

Fig. 11 shows an embodiment in which the present invention is applied to an automotive air-conditioning system with an odor sensor installed in the cabin. The entire air flow passage of the automotive air-conditioning system is shown. An inner-outer air change-over box 311 positioned at an end of this air flow passage causes an inner-outer air change-over damper 312 to switch the opening and closing of an inner air vent 313 and an outer air inlet 314. A blower 315 is driven by a motor 316. A cooling heat exchanger 318 includes a refrigerant evaporator in freezing cycles having a compressor driven by an automotive engine. A heater unit case 319 has built therein a heating heat exchanger 320 having a heater core (hot water heat radiator) with the cooling water of the automotive engine as a heat source. This heat exchanger 320 has on the side thereof an air mix damper 322 for regulating the rate of cool air passing through a bypass 321 and the hot air supplied through the heat exchanger 320. By regulating the opening degree of this damper 322, the temperature of air blown into the cabin is regulated as desired. The cool and hot airs are mixed in the downstream of the air mix damper 322 for regulating the rate of cool air passing through a bypass 321 and the hot air supplied through the heat exchanger 320. By regulating the opening degree of this damper 322, the temperature of air blown into the cabin is regulated as desired. The cool and hot airs are intermixed in the downstream of the heat exchanger 320. Numeral 324 designates a damper for switching upward and downward blowouts, and 325 a downward blowout vent for blowing out air downward at the feet of the occupants. 326 designates an upward blowout duct, and 327 an upward blowout vent disposed downstream of the blowout duct 326. The vent 327 is mounted demountably above the front side of the instrument panel 328 of the automobile. 329 designates a wind direction changer rotatably mounted in the upward blowout vent 327, and 330 a deodorizer arranged immediately upstream of the upward blowout vent 327 within the duct 326. Normally, the deodorizer 330 is accommodated in parallel to the air flow in a recess 331 formed integrally with the duct 326. This deodorizer 330 has a replaceable filter member of a resin or the like held demountably in the duct 326, and is installed rotatably on the duct 326 by a shaft.

The present embodiment uses an odor sensor 360 shown in Fig. 1 lacking a temperature-compensated sensor shown in Embodiment 1. An LB film of phospholipid like recithin, cholesterol, an artificial synthetic film, a cellulose film, arachic acid or the like material is inserted between input and output electrodes of the sensor 360, thereby making it possible to detect the sulfuric compounds like $SO_2$, $H_2S$ and $HSO_3$, $NO_x$, ammonium, exhaust gas and the like.

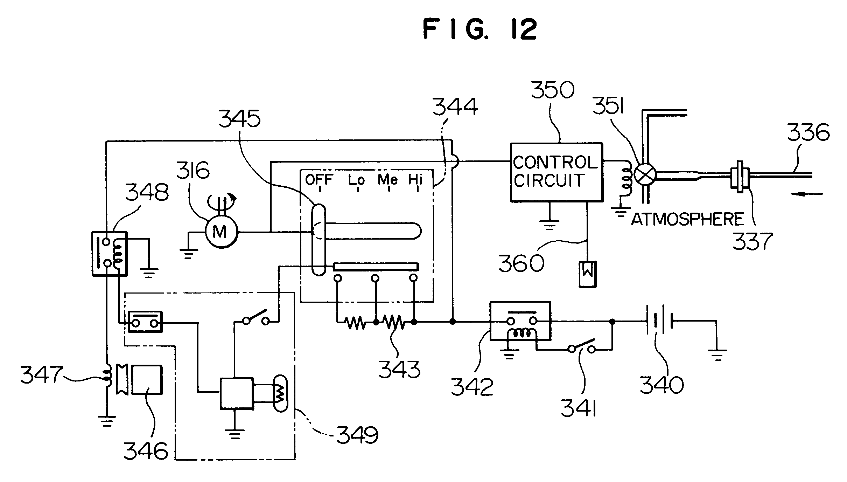

Fig. 12 shows an electrical circuit, including an onboard battery 340, an ignition switch 341 for an onboard engine, a relay 342, a blower speed-change resistor 343, a blower switch 344, a switch movable member 345, a freezing-cycle compressor 346 having an evaporator 318, a magnetic clutch 347 therefor, a relay 348, a compressor control circuit 349 and a control circuit 350 according to the present invention. A signal generated by the odor sensor 360 installed in the interior is used as an input. While the odor sensor 360 detects the odor of a predetermined concentration or higher, the deodorizer 330 is held at a position indicated by one-dot chain in Fig. 11 in such a manner as to cover the upstream side of the upward blowout vent 327 in the duct 326. The odor sensor 360 is adapted to adsorb an odorous substance on the surface thereof by chemical process in gas phase, and detects the weight change of the gas component on the surface thereof from a frequency change.

In the case where the odor sensor 360 installed at a position of an indoor room lamp in Fig. 12 fails to detect the odor of more than a predetermined concentration, electric power supply to the blower motor 316, the control circuit 350, etc. are entirely cut off, and therefore the air-conditioning system is de-energized. Under this condition, the solenoid valve 351 is turned off and communicates with a negative-pressure diaphragm 337. The deodorizer 330 is rotated up to the point of solid line and is held at a position parallel to the air passage in the duct 3267.

Next, assume that the ignition switch 341 is thrown on to turn on the relay 342, with the blower switch 345 turned on at a low speed (Lo), medium speed (Me) or high speed (Hi). The blower motor 316 is energized, and the blower 315 starts thereby to activate the air-conditioning system. At the same time, the control circuit 350 is energized. In the case where the signal from the sensor 360 is not detected, however, the control unit 350 does not energize the solenoid valve 351, with the result that the deodorizer 330 remains inoperative at a position parallel to the air passage.

In the case where an odor component entering from outside the vehicle is sensed by the odor sensor 360, on the other hand, the control circuit 350 moves the deodorizer 330 to a position crossing the air flow shown by one-dot chain in Fig. 11, followed by the deodorizer removing the offensive odor thereby to prevent the occupants from feeling uncomfortable.

When the blower switch 344 is turned off, the solenoid valve 351 is de-energized, and the negative-pressure diaphragm 337 is supplied with atmospheric air. Therefore, the deodorizer 330 is restored to the position of solid line in preparation for detection of an offensive odor. The deodorizing filter is not limited to activated carbon, but may use a chemical adsorbent like an activated carbon which suports various metal ions or metal catalysts in accordance with the components to be eliminated from an offensive odor, or an aromatic deodorizing agent attached thereto.

According to the present embodiment, an offensive odor component discharged with a blown-out air into the room is eliminated in satisfactory manner by a deodorizer. In view of the fact that the blown-out air is not applied through a deodorizer when no odor is generated, the wind resistance is not increased, with the result that the desired air-conditioning ability is exhibited without increasing the capacity of the blower motor.

Further, the physical odors of cigarette smoke or amine components or a strong smell of cosmetics in the cabin, or an offensive odor of putrid or musty foods composed of sulfuric or amine components is detected. In the case where these odors exceed a tolerance, a device for self cleaning is operated. A system thus operated to energize a deodorizing or odor-eliminating device realizes a comfortable living space or vehicle environment. A system with an air-conditioner, a car cooler or an air freshener operated by a self-cleaning device is equipped with a system for notifying the required time of cleaning.

Embodiment 5

In a dish washer, when a start button in the control section is depressed with tableware placed in a basket, water is supplied from a shower nozzle to wash dishes and other tableware. A pump is operated and air blown out and sprayed from an air vent to dry the dishes. After the washing process for a predetermined length of time, stained components of protein and lipids are detected by a sensor shown in Fig. 1 of Embodiment 1 with an artificial synthetic enzyme film installed in the bath body inside the cover. On the basis of the resulting detection value, the amount of water and detergent, and the time lengths of washing, rinsing and drying are automatically determined to finalize the washing processes.

In a dish washer, the problem is posed by garbage remaining after the machine use which generates an offensive odor or a putrid smell of protein or sulfuric components of hydrogen sulfide, indole and methyl mercaptan decomposed from the putrid smell of protein, or an odor of fish or putrid fish decomposed as amine components of ammonium, ethyl amine and trimethyl amine. These offensive odors are detected by a sensor configured as described above installed on the interior of the cover except when dishes are being washed, so that when a tolerance is exceeded, the resulting signal is used to indicate on a control panel or a deodorizer or an odor-eliminator is energized automatically. In the process, a molecular film having liposome is used for a sensing portion of the sensor.

Embodiment 6

In a full-automatic washing machine, cloths and detergent are placed in a washing bath. When a start button on the control panel is depressed, water is supplied and the motor started to rotate the washing bath and rotative blades. The washing process is thus conducted and waste water discharged from the drain hose. In such a full-automatic washer, the problem is that mold grows due to residual flocks and soap water. Thus an odor sensor shown in Fig. 1 of Embodiment 1 is installed at the upper part inside the washer body covering the washing bath or outside the washing bath, control panel or the cover to detect the musty odor of the washing bath or the whole body of the automatic washing machine. In the case where the detection value exceeds a tolerance, the advisability of cleaning is notified by a display unit installed on the control panel.

In similar fashion, an odor sensor is installed on the inside of a control panel or a washing bath, and the odor emanating from cloth stains is detected. The amount of water and detergent and the time lengths of washing, rinsing and drying are automatically determined to perform a full-automatic washing operation. In the process, a molecular film having liposome is arranged on the sensing portion of the sensor.

Further, in the case where an enzyme detergent is used, an odor sensor is installed on the interior of the washing bath, and the reduction rate of enzyme consumed corresponding to cloth stains is measured. The enzyme detergent is thus automatically added, and the time of washing, rinsing and drying are determined automatically thereby making possible a full-automatic washing process. The sensing portion of the sensor is equipped with an enzyme film reactive to the enzyme detergent.

Embodiment 7

In a cloth dryer, wet clothes are placed in a drum, into which a hot air is sent by the operation of a heater, a heat exchanger and a motor. The clothes are then rotated and the water content of the clothes is collected in a duct and discharged through a drain hose. The problem of the cloth dryer is the offensive odor generated from the sweat remaining on the clothes and residual cosmetics left uncleaned. A sensor is therefore installed on the inside of the door or a flocks pocket to detect the offensive odor. As an alternative method, a display unit indicating detection values in several stages is inserted to determine the advisability of rewashing. For this purpose, a molecular film having liposome is formed on the sensing portion of the sensor.

Embodiment 8

The sensor according to the present invention permits selection of a reactive material installed on a sensing portion, whereby an intended substance can be detected selectively with rapidity and high sensitivity. If a sensor according to the present invention is installed with an analytical system for analyzing $NO_x$ or $SO_x$, therefore, rapid and highly-sensitive analysis of a specific component is made possible.

Embodiment 9

A sensor according to the present invention is installed on a clinical chemical analyzer to conduct rapid and highly sensitive analysis of specific biological productions. For this purpose, the sensing portion of the sensor has an enzyme film fixed. If this enzyme is formed of glucose oxidase, alcohol oxidase, glucerol dehydrogenase, lactate oxidase, uricase, glutamate dehydrogenase, asparaginase, methionine ammonialyase, urease, cholesterol oxidase, lipase, phospholipase, pennicilinase, glyatinase or phosphatase, then the biological components of glycose, ethanol, glycerol, lactic acid, uric acid, glutamic acid, asparagin, methionine, urea, cholesterol, neutral lipid, phosphor lipid, pennicilin, creatin or phosphoric ions can be selectively detected respectively. It is thus possible to provide a clinical chemical analysis system for controlling the health of human bodies.

According to the present invention, a minute amount of reaction or a minute component of a metal, ceramics or an organic chemical compound in a gas-phase environment is detected with high sensitivity in the order of ng. The present invention is thus applicable to a biological component sensor of a clinical chemical analysis system in a field involving the problem of reaction of a very small amount, a corrosion detection sensor as a probe for reliability evaluation of electronics and information processing systems, or a humidity sensor, a gas sensor, an odor sensor, a bacteria sensor or a minute ion concentration sensor. The reliability of these devices is thus further improved. Also, by utilizing this sensor for various home-use electric appliances or air-conditioners, various devices incorporated therein are capable of being controlled, thereby making it possible to conduct life more comfortably. Further, the piezoelectric substrate according to the present invention is high in mass productivity, and is so small in size that a system lower in cost and smaller in size is made possible.

A Japanese Patent Application Hei 3-71452 filed April 4, 1992 and JP-A-3-115853 are hereby incorporated herein by reference.

The present invention has been described in detail, it should be understood that various changes, substitutions and alternations can be made hereto without departing from the spirit and scope of the present invention as defined by the appended claims.

## Claims

1. A method of detecting a minute gas component in an ambience comprising the steps of:

   applying an ultrasonic vibration to a thin film (4) mounted on a piezoelectric substrate (2); and detecting the minute gas component by detecting a frequency change propagated through said thin film (4) due to the reaction between said thin film (4) and the minute gas component in the ambience.

2. A method of monitoring an environment comprising the steps of:

   applying an ultrasonic vibration to a thin film (4) mounted on a piezoelectric substrate (2); and measuring the corrosive behaviour affecting the material constituting said thin film (4) in an ambience by detecting the frequency change propagating through said thin film (4) due to the reaction between said thin film (4) and said ambience.

3. A method of monitoring an environment comprising the steps of:

   applying an ultrasonic vibration to a thin film (4) mounted on a piezoelectric substrate (2); and detecting the change in quality of the material constituting said thin film (4) in an ambience by detecting the frequency change propagating through said thin film (4) due to the reaction between said thin film (4) and the ambience.

4. A method of monitoring an environment comprising the steps of; detecting a minute gas component in an ambience or a weight change of a material constituting a thin film (4) mounted on a piezoelectric substrate (2) in said ambience by applying an ultrasonic vibration to said thin film (4); and

   detecting the frequency change propagating through said thin film (4) due to the reaction between said thin film (4) and said ambience, wherein said thin film includes at least one of a metal, an organic chemical compound and ceramics.

5. An apparatus comprising a detection device for detecting a minute gas component in an ambience, wherein said gas detection device includes a gas sensor (1) for sensing said minute gas component, oscillation means (133) for applying an ultrasonic vibration to said gas sensor (1) and detector means for detecting the frequency of a surface acoustic wave vibration generated from said gas sensor, and said gas sensor includes a piezoelectric substrate (2), a thin film (4) composed of a material reactive to said minute gas component mounted on said substrate surface, an electrode (3, 3') mounted on said piezoelectric substrate for applying said ultrasonic vibration and an electrode (3, 3') for detecting said ultrasonic vibration thus applied.

6. An apparatus according to Claim 5, wherein said gas detection device further including display means (138) for displaying the contents of the minute gas component in accordance with the quantity thereof from a signal produced from said detector means.

7. An apparatus comprising an odor detection device (132) for detecting an offensive component in an ambience, wherein said odor detection device includes a gas sensor (1) for sensing said odor, oscillation means (133) for causing an ultrasonic vibration in said gas sensor, and detector means for detecting the frequency of the surface acoustic wave generated from said gas sensor, and said gas sensor includes a piezoelectric substrate (2), a thin film (4) composed of a material reactive to the components of said odor mounted on said substrate surface, an electrode (3, 3') mounted on said substrate surface for applying said surface acoustic wave and an electrode (3, 3') for detecting said surface acoustic wave thus applied.

8. An apparatus comprising an odor detection device (132) according to Claim 7, said odor detection device further including display means (138) for displaying the contents of the odor component from said detector means in accordance with the signal output corresponding to said odor component or a deodorizing device for removing said odor component in accordance with said output.

9. An apparatus comprising a gas detection device (132) for detecting a corrosive environment of an ambience, wherein said gas detection device includes a gas sensor (1) reactive to said ambience, oscillation means (133) for applying an ultrasonic vibration to said gas sensor and detection means for detecting the frequency of the surface acoustic wave generated from said gas sensor, and said gas sensor includes a piezoelectric substrate (2), a thin film (4) composed of a metal mounted on said piezoelectric substrate and reactive to said ambience, an electrode (3, 3') mounted on said piezoelectric substrate for causing said ultrasonic vibration and an electrode (3, 3') for detecting said ultrasonic vibration thus caused.

10. A refrigerator comprising a heat insulating material (207) filled between an outer box (202) and an inner box (203), a refrigeration chamber (209) and a freezing chamber (208) partitioned in said inner box, wherein at least said refrigeration chamber includes a gas sensor (1) therein for detecting the putrid smell of foods and display means (138) for displaying the degree of putridity in accordance with the output of said gas sensor.

11. A refrigerator comprising a heat insulating material (207) filled between an outer box (202) and an inner box (203), a refrigeration chamber (209) and a freezing chamber (208) partitioned in said inner box and an odor detection device (132) for detecting the putrid smell of foods, wherein said odor detection device includes at least a gas sensor (1) mounted in said refrigerator for sensing the odor component, oscillation means (133) for causing an ultrasonic vibration in said gas sensor and detection means (138) for detecting the frequency of the surface acoustic wave generated from said gas sensor, said gas sensor including a piezoelectric substrate (2), a thin film (4) mounted on said substrate surface and composed of a material reactive to said odor component, and an electrode (3, 3') mounted on said piezoelectric substrate for causing the surface acoustic wave and an electrode for detecting said ultrasonic surface acoustic wave, said refrigerator further comprising a display unit for displaying in accordance with the output from said detector means.

12. An air-conditioning system for detecting a minute offensive odor in the indoor air by an odor sensor and replacing with an outside air or deodorizing said indoor air in accordance with the value thus detected, wherein said odor sensor (360) includes a piezoelectric substrate (2) and a thin film (4) mounted on said substrate surface and composed of a material reactive to said odor component, said offensive odor being detected by applying an ultrasonic vibration to said odor sensor (360) from an oscillator (133) and detecting the frequency change of the surface acoustic wave due to the reaction between said thin film (4) and the odor component by a detector.

**13.** A detection device for a minute gas, comprising:

a gas sensor (1) for sensing said minute gas component, oscillation means (133) for applying an ultrasonic vibration to said gas sensor (1) and detector means for detecting the frequency of a surface acoustic wave vibration generated from said gas sensor, and said gas sensor includes a piezoelectric substrate (2), a thin film (4) composed of a material reactive to said minute gas component mounted on said substrate surface, an electrode (3, 3') mounted on said piezoelectric substrate for applying said ultrasonic vibration and an electrode (3, 3') for detecting said ultrasonic vibration thus applied.

**14.** A detection device for a minute chemical, comprising:

a sensor (1) for sensing said minute chemical, oscillation means (133) for applying an ultrasonic vibration to said gas sensor (1) and detector means for detecting the frequency of a surface acoustic wave vibration generated from said gas sensor, and said gas sensor includes a piezoelectric substrate (2), a thin film (4) composed of a material reactive to said minute chemical mounted on said substrate surface, an electrode (3, 3') mounted on said piezoelectric substrate for applying said ultrasonic vibration and an electrode (3, 3') for detecting said ultrasonic vibration thus applied.

**15.** A detection device according to claim 14, further comprising:

means for compensating the temperature change of the substrate.

# F I G. I

# F I G. 2

# F I G. 3

# F I G. 4

RH95%, 30°C

TIME (sec)

# F I G. 5

# F I G. 6

# F I G. 7

# F I G. 8

# FIG. 9

# FIG. 10

FIG. 11

# F I G. 12

EP 0 509 328 A2